# EUROPÄISCHE PATENTANMELDUNG

(11) **EP 0 080 103 A1**
(43) Veröffentlichungstag der Anmeldung: **01.06.1983**
(21) Anmeldenummer: 82110297.7
(22) Anmeldetag: 09.11.1982
(51) Int. Cl.: C07D 249/08, C07D 233/54, C07D 233/60, C07D 409/06, A01N 43/50, A01N 43/64

(54) **Azolylvinyldithioacetale, Verfahren zu ihrer Herstellung und ihre Verwendung als Fungizide**

(30) Priorität: 19.11.1981 DE 3145890
(71) Anmelder: BAYER AG, 51368 Leverkusen (DE)
(72) Erfinder: Kraatz, Udo, Dr., D-5090 Leverkusen 1 (DE); Jäger, Gerhard, Dr., D-5090 Leverkusen 1 (DE); Büchel, Karl Heinz, Prof. Dr., D-5093 Burscheid (DE); Frohberger, Paul-Ernst, Dr., D-5090 Leverkusen 1 (DE)

(57) **Zusammenfassung**

Neue Azolylvinyldithioacetale der Formel 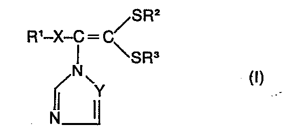 in welcher
R¹ für Alkyl, gegebenenfalls substituiertes Cycloalkyl, gegebenenfalls substituiertes Phenyl oder Alkoxy steht,
R² und R³ gleich sind und für Alkyl, Alkenyl, Alkinyl, gegebenenfalls substituiertes Benzyl oder Trialkylsilyl stehen, oder
R² und R³ gemeinsam für eine Alkylenkette, für eine Dialkylsilylbrücke oder die -CH = CH-Gruppe stehen,
X für die Ketogruppe oder die CH-(OH)-Gruppe steht und
Y für ein Stickstoffatom oder die CH-Gruppe steht, sowie deren Säureadditions-Salze und Metallsalz-Komplexe, ein Verfahren zur Herstellung dieser neuen Stoffe und deren Verwendung als Fungizide.

## Beschreibung

Die vorliegende Erfindung betrifft neue Azolylvinyldithioacetale, ein Verfahren zu ihrer Herstellung und ihre Verwendung als Fungizide.

Es ist bereits bekannt geworden, daß bestimmte Triazolylalkenone und -ole gute fungizide Eigenschaften aufweisen (vgl. DE-OS 29 29 602). So lassen sich beispielsweise 4,4-Dimethyl-1-(naphth-2-yl)-2-(1,2,4-triazol-1-yl)-1-penten-3-on und 4,4-Dimethyl-1-(naphth-2-yl)-2-(1,2,4-triazol-1-yl)-1-penten-3-ol zur Bekämpfung von Pilzen einsetzen. Die Wirkung dieser Verbindungen ist jedoch, insbesondere bei niedrigen Aufwandmengen und -konzentrationen, nicht immer ganz befriedigend.

Es wurden nun neue Azolylvinyldithioacetale der Formel 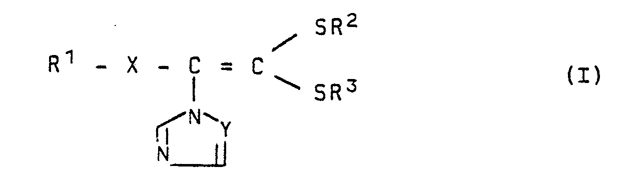 in welcher
R für Alkyl, gegebenenfalls substituiertes Cycloalkyl, gegebenenfalls substituiertes Phenyl oder Alkoxy steht,
_{R}² und _{R}³ gleich sind und für Alkyl, Alkenyl, Alkinyl, gegebenenfalls substituiertes Benzyl oder Trialkylsilyl stehen, oder
R² und R³ gemeinsam für eine Alkylenkette, für eine Dialkylsilylbrücke oder die -CH=_{CH}-Gruppe stehen,
X für die Ketogruppe oder die CH (OH)-Gruppe steht und
Y für ein Stickstoffatom oder die CH-Gruppe steht,

sowie deren Säureadditions-Salze und Metallsalz-Komplexe gefunden.

Weiterhin wurde gefunden, daß man die Azolylvinyldithioacetale der Formel (I) sowie deren Säureadditions-Salze und Metallsalz-Komplexe erhält, wenn man α-Azolyl-ketone der Formel 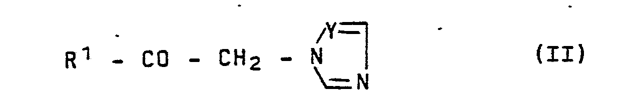 in welcher
R¹ und Y die oben angegebene Bedeutung haben,

zunächst mit Schwefelkohlenstoff in Gegenwart einer Base und in Gegenwart eines Verdünnungsmittels versetzt und anschließend entweder
α) mit einer Verbindung der Formel 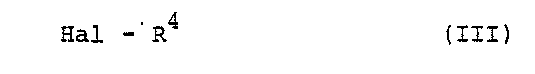 in welcher
   Hal für Halogen steht und
   _{R}⁴ für Alkyl, Alkenyl, Alkinyl, gegebenenfalls substituiertes Benzyl oder Trialkylsilyl steht,

   oder
ß) mit Dimethylsulfat der Formel 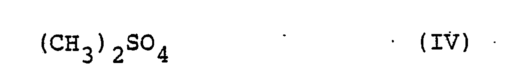 oder
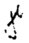 ) mit einem Dihalogenid der Formel 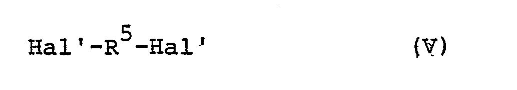 in welcher
   Hal' für Halogen steht und
   _{R}⁵ für eine ein- oder mehrgliedrige Methylenkette, für eine Dialkylsilylbrücke oder eine -CH=CH-gruppe steht,

   in Gegenwart des gleichen Verdünnungsmittels umsetzt; und gegebenenfalls die erhaltenen Keto-Derivate der Formel 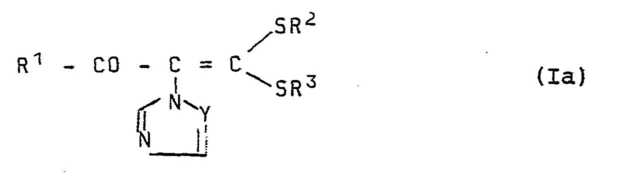 in welcher
   _{R}¹, _{R}², _{R}³ und _{Y} die oben angegebene Bedeutung haben,

   in allgemein üblicher Weise reduziert;
   .und gegebenenfalls anschließend an die so erhaltenen Verbindungen der Formel (I) eine Säure oder ein Metallsalz addiert.

Schließlich wurde gefunden, daß die neuen Azolylvinyldithioacetale der Formel (I) sowie deren Säureadditions-Salze und Metallsalz-Komplexe starke fungizide Eigenschaften besitzen.

Überraschenderweise zeigen die erfindungsgemäßen Stoffe eine bessere fungizide Wirksamkeit als die aus dem Stand der Technik bekannten Verbindungen 4,4-Dimethyl-1-(naphth-2-yl)-2-(1,2,4-triazol-1-yl)-1-penten-3-on und 4,4-Dimethyl-1-(naphth-2-yl)-2-(1,2,4-triazol-1-yl)-1-penten-3-ol, welche wirkungsmäßig ähnliche Verbindungen sind.

Die erfindungsgemäßen Azolylvinyldithioacetale sind durch die Formel (I) allgemein definiert. In dieser Formel stehen vorzugsweise
_{R}¹ für geradkettiges oder verzweigtes Alkyl mit 1 bis 4 Kohlenstoffatomen, gegebenenfalls durch Alkyl mit 1 bis 4 Kohlenstoffatomen substitutiertes Cycloalkyl mit 3 bis 7 Kohlenstoffatomen, geradkettiges oder verzweigtes Alkoxy mit 1 bis 4 Kohlenstoffatomen, sowie für gegebenenfalls substitutiertes Phenyl, wobei als Substituenten genannt seien: Halogen; Alkyl, Alkoxy und Alkylthio mit jeweils 1 bis 4 Kohlenstoffatomen; Halogenalkyl, Halogenalkoxy und Halogenalkylthio mit jeweils 1 bis 2 Kohlenstoff-und bis zu 5 gleichen oder verschiedenen Halogenatomen, wie insbesondere Fluor- und Chloratomen, sowie gegebenenfalls durch Halogen und/oder Alkyl mit 1 bis 2 Kohlenstoffatomen substituiertes Phenyl und Phenoxy;
_{R}² und _{R}³ für den gleichen Rest, wie geradkettiges oder verzweigtes Alkyl mit 1 bis 4 Kohlenstoffatomen, geradkettiges oder verzweigtes Alkenyl und Alkinyl mit jeweils 2 bis 6 Kohlenstoffatomen, gegebenenfalls substituiertes Benzyl, wobei als Substituenten am Phenylring der Benzylgruppe in Frage kommen: Halogen; Alkyl, Alkoxy und Alkylthio mit jeweils 1 bis 4 Kohlenstoffatomen; Halogenalkyl, Halogenalkoxy und Halogenalkylthio mit jeweils 1 bis 2 Kohlenstoff- und bis zu 5 gleichen oder verschiedenen Halogenatomen, wie insbesondere Fluor- und Chloratomen, sowie gegebenenfalls durch Halogen und/oder Alkyl mit 1 bis 2 Kohlenstoffatomen substituiertes Phenyl und Phenoxy; und weiterhin stehen vorzugsweise
R2 und _{R}³ jeweils für _{T}rialkylsilyl mit 1 bis 4 Kohlenstoffatomen in jedem Alkylteil;
R² und _{R}³ gemeinsam für eine Alkylenkette mit 1 bis 4 Kohlenstoffatomen, für eine Dialkylsilylbrücke mit 1 bis 4 Kohlenstoffatomen in jedem Alkylteil oder für die -CH=_{CH}-Gruppe,
X für die Ketogruppe oder die-CH(OH)-Gruppe und
Y für ein Stickstoffatom oder eine CH-Gruppe.

Besonders bevorzugt sind diejenigen Verbindungen der Formel (I), in denen
_{R}¹ für tert.-Butyl, Isopropyl; gegebenenfalls durch Methyl, Ethyl oder Propyl substituiertes Cyclopropyl, Cyclopentyl oder Cyclohexyl; Methoxy, Ethoxy, Butoxy; sowie für gegebenenfalls substituiertes Phenyl steht, wobei als Substituenten genannt seien: Fluor, Chlor, Methyl, Isopropyl, tert.-Butyl, Methoxy, Methylthio, Isopropoxy, Trifluormethyl, Trifluormethoxy, Trifluormethylthio, sowie gegebenenfalls durch Fluor, Chlor und/oder Methyl : substituiertes Phenyl oder Phenoxy;
R² und R³ gleich sind und für geradkettiges oder verzweigtes Alkyl mit 1 bis 4 Kohlenstoffatomen, Allyl, Propargyl oder gegebenenfalls substituiertes Benzyl stehen, wobei als Substituenten am Phenylring der Benzylgruppe genannt seien: Fluor, Chlor, Methyl, Isopropyl, tert.-Butyl, Methoxy, Methylthio, Isopropoxy, Trifluormethyl, Trifluormethoxy, Trifluormethylthio sowie gegebenenfalls durch Fluor, Chlor und/oder Methyl substituiertes Phenyl oder Phenoxy, oder
R² und _{R}³ gleich sind und für Trimethylsilyl stehen,

oder
R² und R 3 gemeinsam für Methylen, eine Dimethylen-oder Trimethylenkette, eine Dimethylsilylbrücke oder eine -CH=CH-Gruppe stehen,
X für die Ketogruppe oder eine -CH(OH)-Gruppe steht und
Y für ein Stickstoffatom oder eine CH-Gruppe steht.

Bevorzugte erfindungsgemäße Verbindungen sind auch Additionsprodukte aus Säuren und denjenigen Azolylvinyldithioacetalen der Formel (I), in denen die Subsituenten _{R}¹_{1 R}², _{R}³,_{X} und Y die Bedeutungen haben, die bereits vorzugsweise für diese Reste genannt wurden.

Zu den Säuren die addiert werden können, gehören vorzugsweise Halogenwasserstoffsäuren, wie z.B. die Chlorwasserstoffsäure und die Bromwasserstoffsäure, insbesondere die Chlorwasserstoffsäure, ferner Phosphorsäure, Salpetersäure, Schwefelsäure, mono- und bifunktionelle Carbonsäuren und Hydroxycarbonsäuren, wie z.B. Essigsäure, Maleinsäure, Bernsteinsäure, Fumarsäure, Weinsäure, Salizylsäure, Sorbinsäure und Milchsäure, sowie Sulfonsäure, wie z.B. p-Toluolsulfonsäure und 1,5-Naphthalindisulfonsäure.

Außerdem bevorzugte erfindungsgemäße Verbindungen sind Additionsprodukte aus Salzen von Metallen der II. bis IV. Haupt- und der I. und II. sowie IV. bis VIII. Nebengruppe und denjenigen Azolylvinyldithioacetalen der Formel (I), in denen die Substituenten R¹, R², R³, X und Y die Bedeutungen haben, die bereits vorzugsweise für diese Reste genannt wurden. Hierbei sind Salze des Kupfers, Zinks, Mangans, Magnesiums, Zinns, Eisens und des Nickels besonders bevorzugt. Als Anionen dieser Salze kommen solche in Betracht, die sich von solchen Säuren ableiten, die zu physiologisch verträglichen Additionsprodukten führen. Besonders bevorzugte derartige Säuren sind in diesem Zusammenhang die Halogenwasserstoffsäure, wie z.B. die Chlorwasserstoffsäure und die Bromwasserstoffsäure, ferner Phosphorsäure, Salpetersäure und Schwefelsäure.

Verwendet man beispielsweise 1,2,4-Triazol-pinakolin, Schwefelkohlenstoff und Dimethylsulfat als Ausgangsstoffe, so kann der Reaktionsablauf nach dem erfindungsgemäßen Verfahren durch das folgende Formelschema wiedergegeben werden:

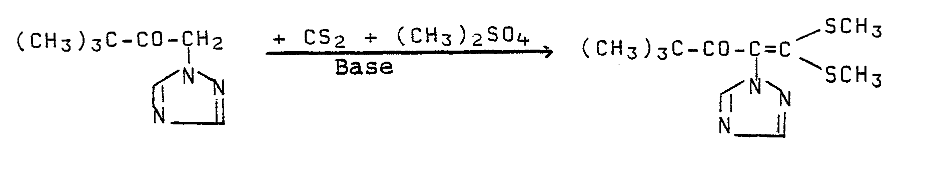

Verwendet man beispielsweise 1,2,4-Triazol-pinakolin, Schwefelkohlenstoff und 1,2-Dibrömethan als Ausgangsstoffe, so kann der Reaktionsablauf nach dem erfindungsgemäßen Verfahren durch das folgende Formelschema wiedergegeben werden:

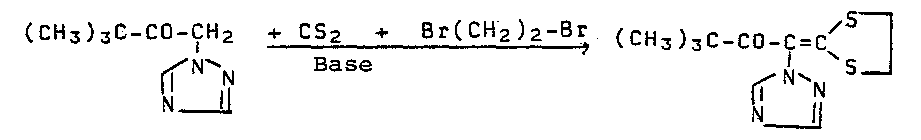

Verwendet man beispielsweise 2,2-Dimethyl-5,5-dime- thylthio-4-(1,2,4-triazol-1-yl)-4-penten-3-on als Ausgangsstoff und Natriumborhydrid als Reduktionsmittel, so kann der Reaktionsablauf nach der erfindungsgemäßen Reduktion durch das folgende.Formelschema wiedergegeben werden:

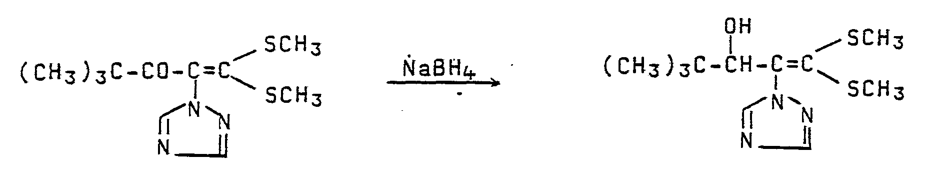

Die bei der Durchführung des erfindungsgemäßen Verfahrens als Ausgangsstoffe benötigten α-Azoiyl-ketone sind durch die Formel (II) allgemein definiert. In dieser Formel stehen R¹ und Y vorzugsweise für die Bedeutungen, die bei der Beschreibung der erfindungsgemäßen Stoffe der Formel (I) bereits vorzugsweise genannt wurden.

Die α-Azolyl-ketone der Formel (II) sind bekannt (vergleiche z.B. DE-OS 24 31 407, 26 38 470 und 30 10 560 sowie JACS 77, 621 (1955)) oder können in allgemein be- .-kannter Art und Weise erhalten werden, indem man die entsprechenden α-Bram(Chlor)-ketone mit Imidazol oder 1,2,4-Triazol in Gegenwart eines inerten organischen Lösungsmittels, wie beispielsweise Aceton, und in Gegenwart eines Säurebindemittels, wie beispielsweise Kaliumcarbonat, vorzugsweise in der Siedehitze umsetzt. Die außerdem für das erfindungsgemäße Verfahren als Reaktionskomponenten zu verwendenden Verbindungen sind durch die Formeln (III), (IV) und (V) definiert. In der Formel (III) steht Hal vorzugsweise für Chlor oder Brom, und R⁴ steht vorzugsweise für die Bedeutungen, die bei der Beschreibung der erfindungsgemäßen Stoffe der Formel (I) bereits vorzugsweise für R² genannt wurden. In der Formel (V) steht Hal' vorzugsweise für Chlor oder Brom, und R⁵ steht vorzugsweise für die Bedeutungen, die bei der Beschreibung der erfindungsgemäßen Stoffe der Formel (I) bereits vorzugsweise für die gemeinsame Substituentenbildung von R² und R 3 genannt wurden.

Die Verbindungen der Formeln (III), (IV) und (V) sind allgemein bekannte Verbindungen der organischen Chemie.

Als Verdünnungsmittel kommen für das erfindungsgemäße Verfahren zur Herstellung der Keto-Derivate der Formel (Ia) unter den Reaktionsbedingungen inerte organische Lösungsmittel in Frage. Hierzu gehören vorzugsweise Ether, wie Tetrahydrofuran oder Dioxan; Alkohole, wie Methanol, Ethanol oder Isopropanol; Amide, wie Dimethylformamid oder Dimethylacetamid; ferner Dimethylsulfoxid, Hexamethylphosphorsäuretriamid oder Sulfolan (Tetrahydrothiophen-1,1-dioxid).

Das erfindungsgemäße Verfahren zur Herstellung der Keto-Derivate der Formel (Ia) wird in Gegenwart einer Base durchgeführt. Hierzu gehören vorzugsweise Alkalihydroxide und -alkoholate, wie Natrium- und Kaliumhydroxid oder Natrium- und Kalium-methylat, -ethylat und -tert.-butylat; Alkalihydride und -amide, wie Natriumhydrid, Natriumamid oder Lithiumisopropylamid, sowie Butyllithium.

Die Reaktionstemperaturen können bei der Durchführung des erfindungsgemäßen Verfahrens zur Herstellung der Keto-Derivate der Formel (Ia) in einem größeren Bereich variiert werden. Im allgemeinen arbeitet man zwischen 0 und 80°C, vorzugsweise zwischen 0 und 60°C.

Bei der Durchführung des erfindungsgemäßen Verfahrens zur Herstellung der Keto-Derivate der Formel (Ia) setzt man auf 1 Mol α-Azolyl-keton der Formel (III) vorzugsweise 1 bis 1,1 Mol Schwefelkohlenstoff und 2 Mol Base sowie 2 Mol einer Verbindung der Formel (III) oder (IV) oder 1 Mol einer Verbindung der Formel (V) ein. Dabei wird vorzugsweise das Keton der Formel (II) vorgelegt, zunächst mit der halben Menge Schwefelkohlenstoff und Base versetzt, anschließend mit der restlichen Menge Schwefelkohlenstoff und Base und zum Schluß mit der jeweiligen Reaktionskomponente der Formel (III), (IV) oder (V). Die Isolierung der Verbindungen der Formel (Ia) erfolgt in üblicher Weise.

Arbeitet man mit komplexen Hydriden, so kommen als Verdünnungsmittel für die erfindungsgemäße Reduktion der Keto-Derivate der Formel (Ia) polare organische Lösungsmittel in Frage. Hierzu gehören vorzugsweise Alkohole, wie Methanol, Ethanol, Butanol, Isopropanol und Ether, wie Diethylether oder Tetrahydrofuran. Diese Reaktion wird im allgemeinen zwischen 0 und 30°C, vorzugsweise zwischen 0 und 20°C durchgeführt. Hierzu setzt man auf 1 Mol eines Ketons der Formel (Ia) etwa 1 Reaktions- äquivalent eines komplexen Hydrids, wie Natriumborhydrid oder Lithiumalanat, ein. Zur Isolierung der reduzierten Verbindungen der Formel (I) wird der Rückstand in verdünnter Salzsäure aufgenommen, anschließend alkalisch gestellt und mit einem organischen Lösungsmittel extrahiert. Die weitere Aufarbeitung erfolgt in üblicher Weise.

Arbeitet man bei der Reduktion der Keto-Derivate der Formel (Ia) mit Aluminiumisopropylat, so kommen als Verdünnungsmittel für die erfindungsgemäße Umsetzung bevorzugt Alkohole, wie Isopropanol, oder inerte Kohlenwasserstoffe, wie Benzol, in Frage. Die Reaktionstemperaturen können wiederum in einem größeren Bereich variiert werden; im allgemeinen arbeitet man zwischen 20 und 120°C, vorzugsweise zwischen 50 und 100°C. Zur Durchführung der Reaktion setzt man auf 1 Mol eines Ketons der Formel (Ia) etwa 1 bis 2 Mol Aluminiumisopropylat ein. Zur Isolierung der reduzierten Verbindungen der Formel (I) wird das überschüssige Lösungsmittel durch Destillation im Vakuum entfernt und die entstandene Aluminiumverbindung mit verdünnter Schwefelsäure oder Natronlauge zersetzt. Die weitere Aufarbeitung erfolgt in üblicher Weise.

Zur Herstellung von Säureadditions-Salzen der Verbindungen der Formel (I) kommen vorzugsweise diejenigen Säuren in Frage, die bereits im Zusammenhang mit der Beschreibung der erfindungsgemäßen Säureadditions-Salze als bevorzugte Säuren genannt wurden.

Die Säureadditions-Salze der Verbindungen der Formel (I) können in einfacher Weise nach üblichen Salzbildungsmethoden, z.B. durch Lösen einer Verbindung der Formel (I) in einem geeigneten inerten Lösungsmittel und Hinzufügen der Säure, z.B. Chlorwasserstoffsäure, erhalten werden und in bekannter Weise, z.B. durch Abfiltrieren isoliert und gegebenenfalls durch Waschen mit einem inerten organischen Lösungsmittel gereinigt werden.

Zur Herstellung von Metallsalz-Komplexen von Verbindungen der Formel (I) kommen vorzugsweise Salze von denjenigen Anionen und Kationen in Betracht, die bereits im Zusammenhang mit der Beschreibung der erfindungsgemäßen Metallsalz-Komplexe als bevorzugt genannt wurden.

Die Metallsalz-Komplexe von Verbindungen der Formel (I) können in einfacher Weise nach üblichen Verfahren erhalten werden, so z.B. durch Lösen des Metallsalzes in Alkohol, z.B. Ethanol und Hinzufügen zur Verbindung der Formel (I). Man kann Metallsalz-Komplexe in bekannter Weise, z.B. durch Abfiltrieren, isolieren undgegebenenfalls durch Umkristallisation reinigen.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Bakterizide Mittel werden im Pflanzenschutz zur Bekämpfung von Pseudomonadaceae, Rhizobiaceae, Enterobacteriaceae, Corynebacteriaceae und Streptomycetaceae eingesetzt.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut und des Bodens.

Als Pflanzenschutzmittel können die erfindungsgemäßen Wirkstoffe mit besonders gutem Erfolg zur Bekämpfung solcher Pilze eingesetzt werden, die echte Mehltauerkrankungen hervorrufen, so zur Bekämpfung von Erysiphe-Arten, wie z.B. gegen den Erreger des Gersten- bzw. Getreidemehltaus (Erysiphe graminis), zur Bekämpfung von Venturia-Arten, wie z.B. gegen den Erreger des Apfelschorfs (Venturia inaequalis), oder zur Bekämpfung von Reiskrankheiten, wie z.B. Pellicularia sasakii. Hervorzuheben ist, daß die erfindungsgemäßen Stoffe auch gute bakterizide Eigenschaften aufweisen.

Die Wirkstoffe können in die üblichen Formulierungen übergeführt werden, wie Lösungen, Emulsionen, Spritzpulver, Suspensionen, Pulver, Stäubemittel, Schäume, Pasten, lösliche Pulver, Granulate, Aerosole, Suspensions-Emulsionskonzentrate, Saatgutpuder, Wirkstoffimprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä. sowie ULV-Kalt-und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als-Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glykol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material, wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäureester, Polyoxyethylen-Fettalkohol-ether, z.B. Alkylarylpolyglykol-ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Vogelfraß, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z.B. durch Gießen, Tauchen, Spritzen, Sprühen, Vernebeln, Verdampfen, Injizieren, Verschlämmen, Verstreichen, Stäuben, Streuen, Trockenbeizen, Feuchtbeizen, Naßbeizen, Schlämmbeizen oder Inkrustieren.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02 Gew.-%, am Wirkungsort erforderlich.

Die Herstellung und die Verwendung der erfindungsgemäßen Wirkstoffe werden durch die nachfolgenden Beispiele veranschaulicht.

### Herstellungsbeispiele

### Beispiel 1

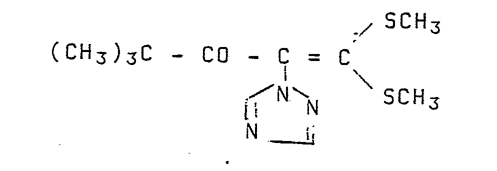

16 g (0,1 Mol) 1,2,4-Triazolyl-pinakolin in 1000 ml Dimethylsulfoxid werden mit 4 g (0,1 Mol) Natriumhydroxid-Pulver versetzt. Dazu werden unter Rühren bei Raumtemperatur 4 g (0,053 Mol) Schwefelkohlenstoff zugetropft. Anschließend wird das Reaktionsgemisch nochmals mit 4 g (0,1 Mol) Natriumhydroxid-Pulver und 4 g (0,053 Mol) Schwefelkohlenstoff versetzt. Man läßt 20 Minuten bei Raumtemperatur nachrühren. Danach werden unter Eiskühlung langsam 25,3 g (0,2 Mol) Dimethylsulfat zugetropft, wobei die Innentemperatur auf ca. 30°C gehalten wird. Nach 3-stündigem Rühren bei Raumtemperatur gießt man das Reaktionsgemisch in Wasser und extrahiert mit Methylenchlorid. Die organische Phase wird getrocknet, eingeengt, und der Rückstand über eine Kieselgelsäule im System Chloroform/Essigester chromatographiert. Man erhält in 53 %iger Ausbeute 2,2-Dimethyl-5,5-dimethylthio-4-(1,2,4-triazol-1-yl)-4-penten-3-on vom Schmelzpunkt 60°C.

### Beispiel 2

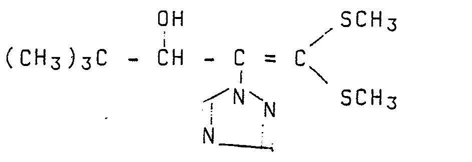

15 g (0,055 Mol) 2,2-Dimethyl-5,5-dimethylthio-4-(1,2,4-triazol-1-yl)-4-penten-3-on (Beispiel 1) werden in 100 ml Methanol gelöst und bei Raumtemperatur mit 2,1 g (0,055 Mol) Natriumborhydrid versetzt. Man läßt 30 Minuten bei 20 bis 30°C nachrühren und engt ein. Der Rückstand wird zwischen Methylenchlorid und Wasser verteilt. Die organische Phase wird abgetrennt, über Natriumsulfat getrocknet und eingeengt. Man erhält 11,9 g (80 % der Theorie) 2,2-Dimethyl-5,5-dimethylthio-4-(1,2,4-triazol-1-yl)-4-penten-3-ol vom Schmelzpunkt 108 - 111°C.

### Beispiel 3

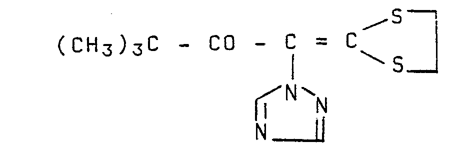

16 g (0,1 Mol) 1,2,4-Triazolyl-pinakolin in 100 ml Dimethylsulfoxid werden mit 4 g (0,1 Mol) Natriumhydroxid-Pulver versetzt. Dazu werden unter Rühren bei .Raumtemperatur 4 g (0,053 Mol) Schwefelkohlenstoff'zugetropft. Anschließend wird das Reaktionsgemisch nochmals mit 4 g (0,1 Mol) Natriumhydroxid-Pulver und 4 g (0,053 Mol) Schwefelkohlenstoff versetzt. Man läßt 20 Minuten bei Raumtemperatur nachrühren. Danach werden unter Eiskühlung 18,8 g (0,1 Mol) 1,2-Dibromethan so zugetropft, daß die Innentemperatur nicht über 30°C steigt. Nach 3-stündigem Rühren bei Raumtemperatur gießt man das Reaktionsgemisch in Wasser. Das ausgefallene Produkt wird abgesaugt und aus Isopropanol umkristallisiert. Man erhält 14,2 g (53 % der Theorie) 3,3-Dimethyl-1-(1,3-dithiolan-2-yliden)-i-(1,2,4-triazol-1-yl)-2-butanon vom Schmelzpunkt 141°C.

In analoger Weise und entsprechend dem erfindungsgemäßen Verfahren werden die in der nachfolgenden Tabelle 1 aufgeführten Verbindungen der Formel (I) erhalten.

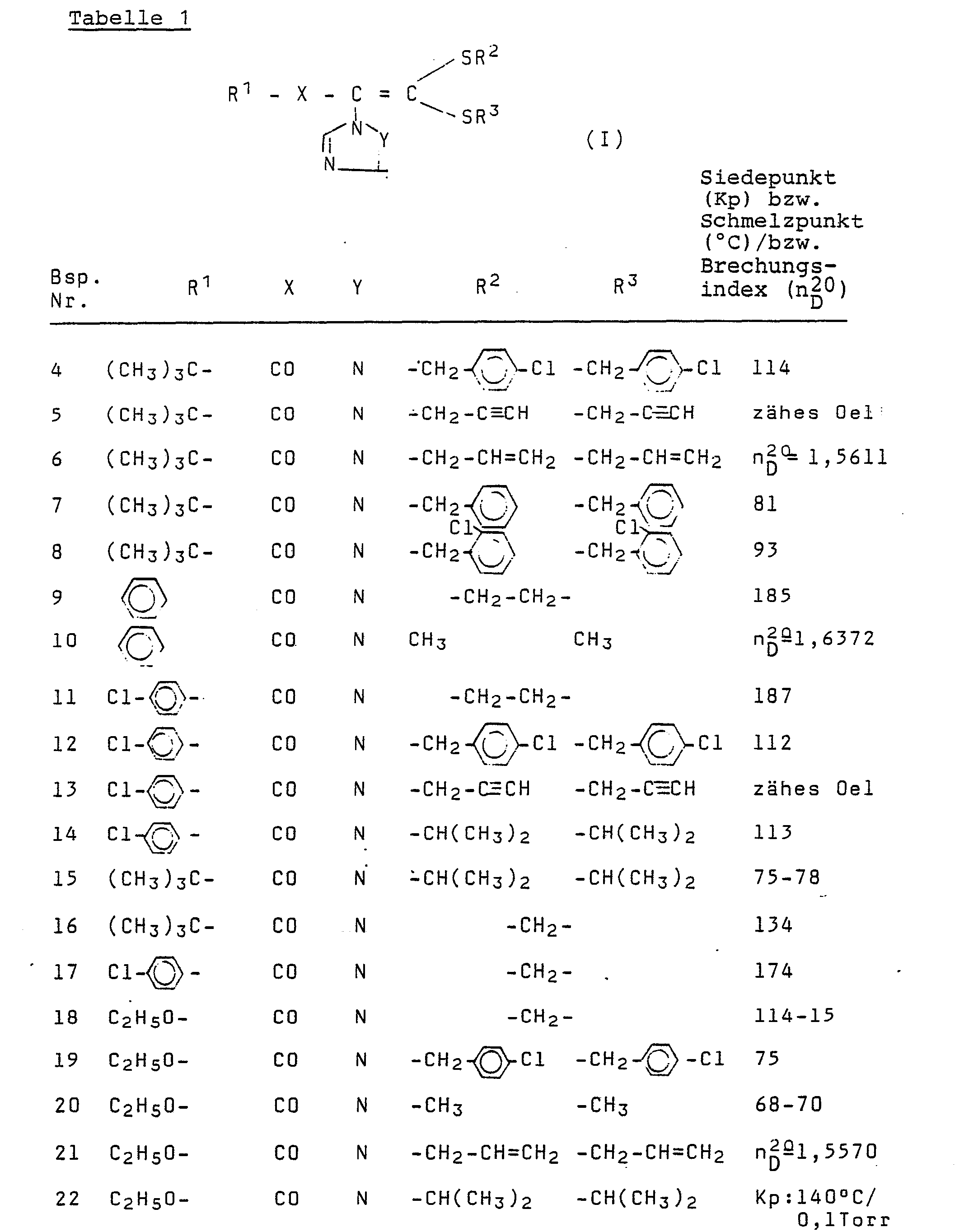

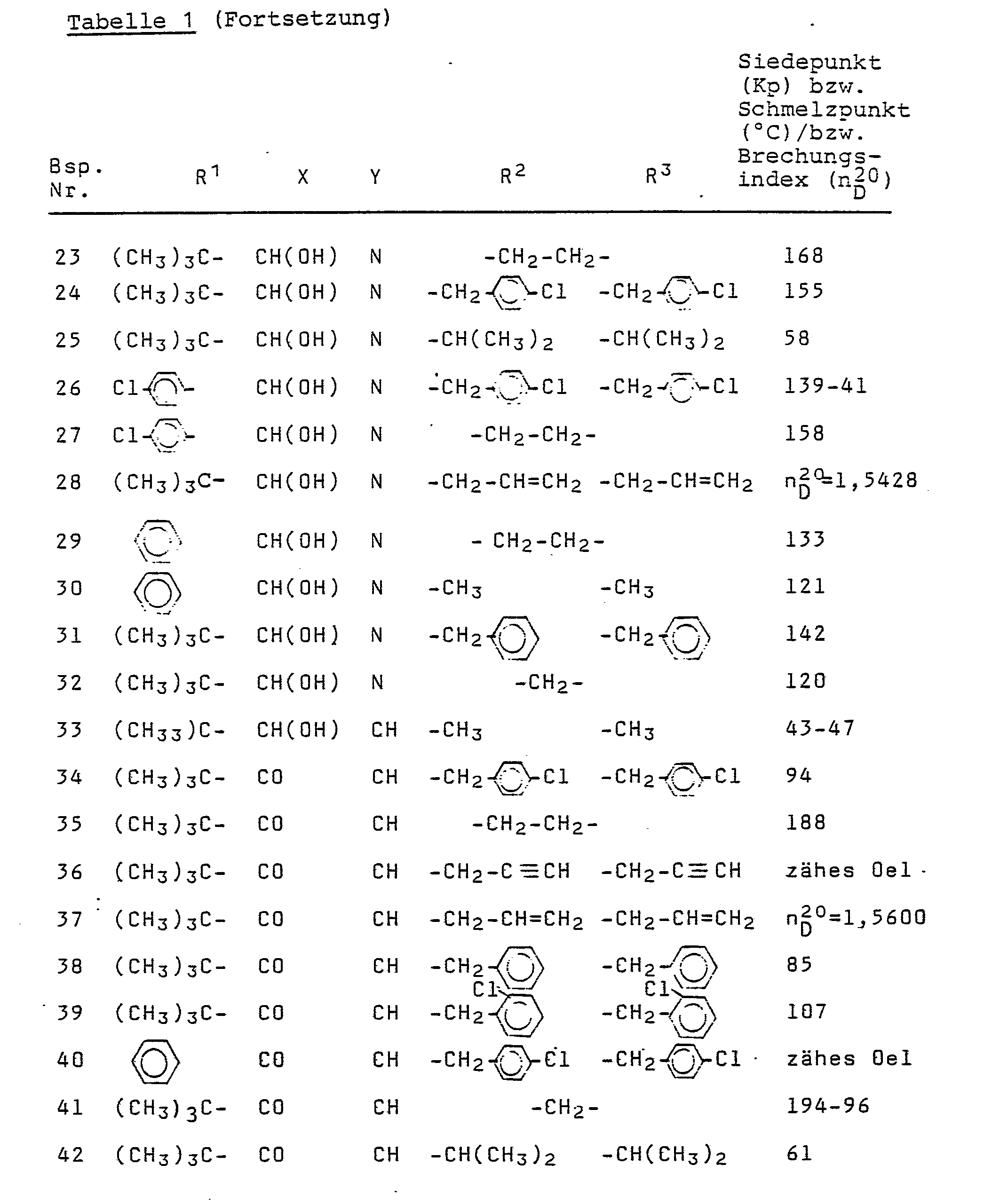

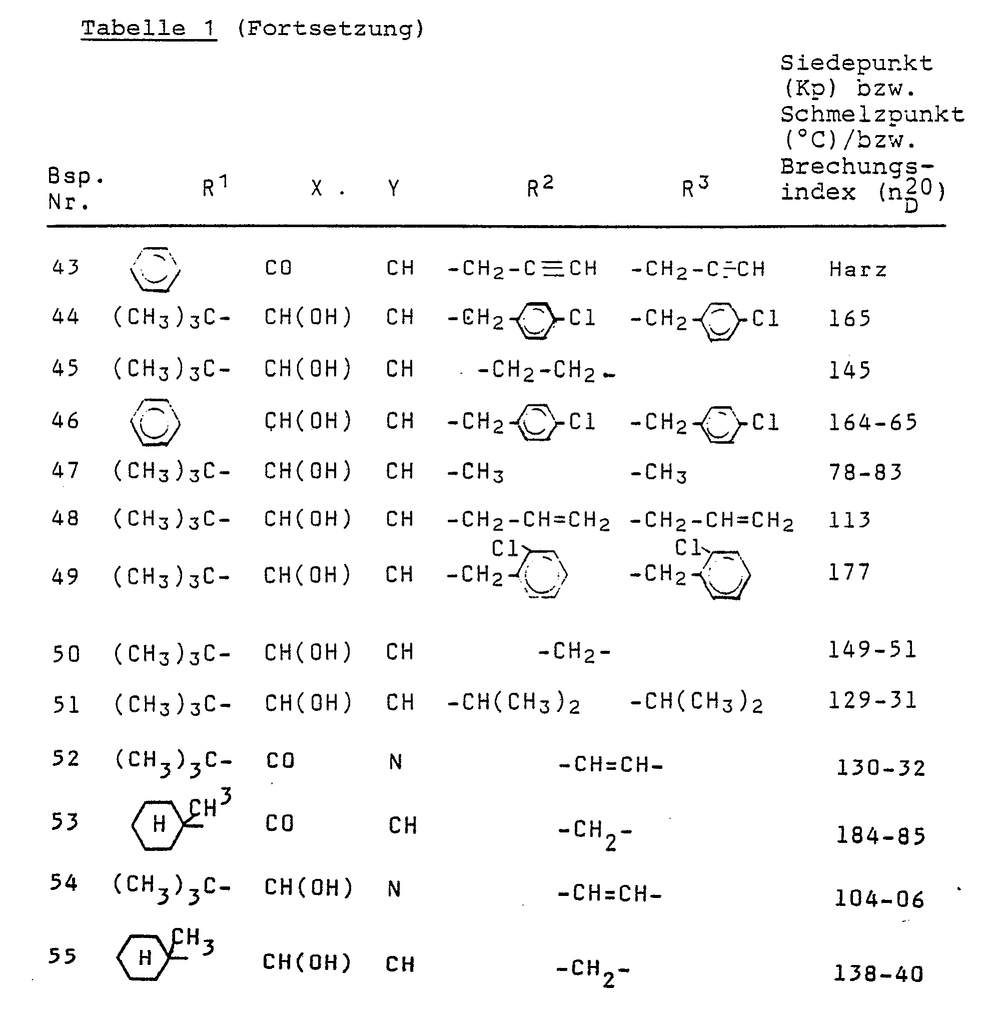

### Verwendungsbeispiele

In den folgenden Beispielen werden die nachstehend angegebenen Verbindungen als Vergleichssubstanzen eingesetzt:

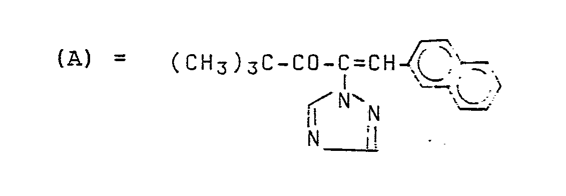

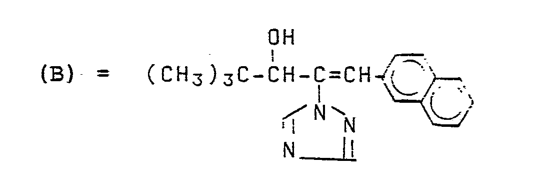

### Beispiel A

### Erysiphe-Test (Gerste) /protektiv

Lösungsmittel: 100 Gewichtsteile Dimethylformamid-Emulgator :0,25 Gewichtsteile Alkylarylpolyglykol-ether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit Sporen von Erysiphe graminis f.sp.hordei bestäubt.

Die Pflanzen werden in einem Gewächshaus bei einer Temperatur von ca. 20°C und einer relativen Luftfeuchtigkeit von ca. 80 % aufgestellt, um die Entwicklung von Mehltaupusteln zu begünstigen.

### 7 Tage nach der Inokulation erfolgt die Auswertung.

Eine deutliche Überlegenheit in der Wirksamkeit gegenüber dem Stand der Technik zeigen bei diesem Test z.B. die. Verbindungen gemäß folgender Herstellungsbeispiele: 38, 7, .8, 42, 19, 22, 45, 51, 2, 47, 28, 48, 25, 32, 27, 29 und 30.

### Beispiel B

### Erysiphe-Test (Gerste) / Saatgutbehandlung

Die Anwendung der Wirkstoffe erfolgt als Trockenbeizmittel. Sie werden zubereitet durch Abstrecken des jeweiligen Wirkstoffes mit Gesteinsmehl zu einer feinpulvrigen Mischung, die eine gleichmäßige Verteilung auf der Saatgutoberfläche gewährleistet.

Zur Beizung schüttelt man das Saatgut 3 Minuten lang mit dem Beizmittel in einer verschlossenen Glasflasche.

Die Gerste sät man mit 3 x 12 Korn 2 cm tief in eine Standarderde. 7 Tage nach der Aussaat, wenn die jungen Pflanzen ihr erstes Blatt entfaltet haben, werden sie mit Sporen von Erysiphe graminis f. sp. hordei bestäubt.

Die Pflanzen werden in einem Gewächshaus bei einer Temperatur von ca. 20°C und einer relativen Luftfeuchtigkeit von ca. 80 % aufgestellt, um die Entwicklung von Mehltaupusteln zu begünstigen.

### 7 Tage nach der Inokulation erfolgt die Auswertung.

Eine deutliche Überlegenheit in der Wirksamkeit gegenüber dem Stand der Technik zeigen bei diesem Test z.B. die Verbindungen gemäß folgender Herstellungsbeispiele: 2, 47, 29.

## Patentansprüche

1) Azolylvinyldithioacetale der Formel 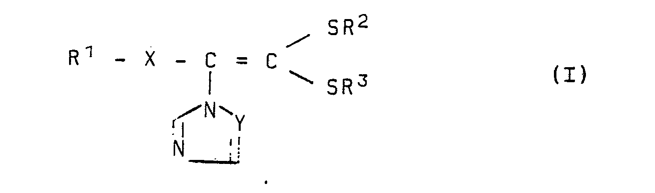 in welcher
R¹ für Alkyl, gegebenenfalls substituiertes Cycloalkyl, gegebenenfalls substituiertes Phenyl oder Alkoxy steht,
_{R}² und R³ gleich sind und für Alkyl, Alkenyl, Alkinyl, gegebenenfalls substituiertes Benzyl oder Trialkylsilyl stehen, oder
_{R}² und R³ gemeinsam für eine Alkylenkette, für eine Dialkylsilylbrücke oder die -CH=CH-Gruppe stehen,
X für die Ketogruppe oder die CH-(OH)-Gruppe steht und
Y für ein Stickstoffatom oder die CH-Gruppe steht,
sowie deren Säureadditions-Salze und Metallsalz-Komplexe.

2) Azolylvinyldithioacetale der Formel (I), in denen
_{R}¹ für geradkettiges oder verzweigtes Alkyl mit 1 bis 4 Kohlenstoffatomen, gegebenenfalls durch Alkyl mit 1 bis 4 Kohlenstoffatomen substituiertes Cycloalkyl mit 3 bis 7 Kohlenstoffatomen, geradkettiges oder verzweigtes Alkoxy mit 1 bis 4 Kohlenstoffatomen oder Phenyl, welches substituiert sein kann*durch Halogen, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 bis 2 Kohlenstoffatomen und bis zu 5 gleichen oder verschiedenen Halogenatomen, Halogenalkoxy mit 1 oder 2 Kohlenstoffatomen und bis zu 5 gleichen oder verschiedenen Halogenatomen, Halogenalkylthio mit 1 oder 2 Kohlenstoffatomen und bis zu 5 gleichen oder verschiedenen Halogenatomen, Phenyl und/ oder Phenoxy, wobei die beiden letztgenannten Substituenten ihrerseits substituiert sein können durch Halogen und/oder Alkyl mit 1 oder 2 . Kohlenstoffatomen,
R² und _{R}³ gleich sind und für geradkettiges oder verzweigtes Alkyl mit 1 bis 4 Kohlenstoffatomen, geradkettiges oder verzweigtes Alkenyl mit 2 bis 6 Kohlenstoffatomen, geradkettiges oder verzweigtes Alkinyl mit 2 bis 6 Kohlenstoffatomen oder für Benzyl stehen, wobei der Phenylteil des Benzylrestes substituiert sein kann durch Halogen, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 bis 2 Kohlenstoffatomen und bis zu 5 gleichen oder verschiedenen Halogenatomen, Halogenalkoxy mit 1 oder 2 Kohlenstoffatomen und bis zu 5 gleichen oder verschiedenen Halogenatomen, Halogenalkylthio mit 1 oder 2 Kohlenstoffatomen und bis zu 5 gleichen oder verschiedenen Halogenatomen, Phenyl und/oder Phenoxy, wobei die beiden letztgenannten Substituenten ihrerseits substituiert sein können durch Halogen und/oder Alkyl mit 1 oder 2 Kohlenstoffatomen,
oder
_{R}² und R³ gleich sind und für Trialkylsilyl mit 1 bis 4 Kohlenstoffatomen in jedem Alkylteil stehen,
oder
R 2 und R³ gemeinsam für eine Alkylenkette mit 1 bis 4 Kohlenstoffatomen, für eine Dialkylsilylbrükke mit 1 bis 4 Kohlenstoffatomen in jedem Alkylteil oder für eine -CH=CH-Gruppe stehen,
X für eine Ketogruppe oder eine CH(OH)-Gruppe steht und
Y für ein Stickstoffatom oder eine CH-Gruppe steht,
sowie deren Säureadditions-Salze und Metallsalz-Komplexe.

3) Verfahren zur Herstellung von Azolylvinyldithioacetalen der Formel 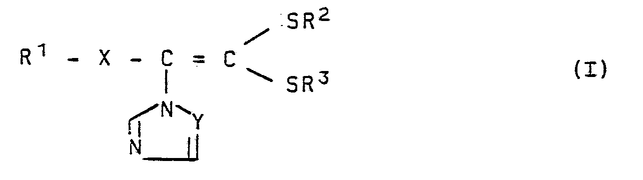 in welcher
R für Alkyl, gegebenenfalls substituiertes Cycloalkyl, gegebenenfalls substituiertes Phenyl oder Alkoxy steht,
_{R}² und _{R}³ gleich sind und für Alkyl, Alkenyl, Alkinyl, gegebenenfalls substituiertes Benzyl oder Trialkylsilyl stehen, oder
R² und R³ gemeinsam für eine Alkylenkette, für eine Dialkylsilylbrücke oder die -CH=CH-Gruppe stehen,
X für die Ketogruppe oder die CH-(OH)-Gruppe steht und
Y für eine Stickstoffatom oder die CH-Gruppe steht,
sowie deren Säureadditions-Salzen und Metallsalz-Komplexen, dadurch gekennzeichnet, daß man α-Azolylketone der Formel 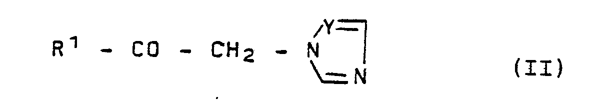 in welcher
R¹ und Y die oben angegebene Bedeutung haben,
zunächst mit Schwefelkohlenstoff in Gegenwart einer Base und in Gegenwart eines Verdünnungsmittels versetzt und anschließend
entweder
α) mit einer Verbindung der Formel 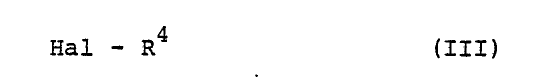
in welcher
Hal für Halogen steht und
_{R}⁴ für Alkyl, Alkenyl, Alkinyl, gegebenenfalls substituiertes Benzyl oder Trialkylsilyl steht, oder
ß) mit Dimethylsulfat der Formel 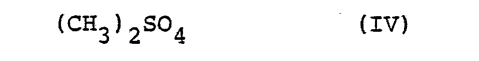
oder
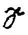 ) mit einem Dihalo^{g}enid der Formel 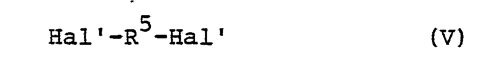 in welcher
Hal' für Halogen steht und
_{R}⁵ für eine ein- oder mehrgliedrige Methylenkette, für eine Dialkylsilylbrücke oder eine -CH=_{CH}-Gruppₑ steht,
in Gegenwart des gleichen Verdünnungsmittels umsetzt;
und gegebenenfalls die erhaltenen Keto-Derivate der Formel 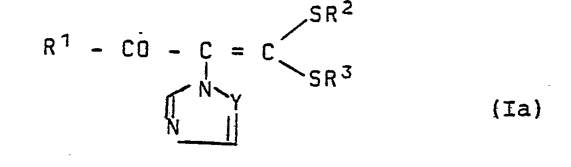 in welcher
_{R}¹, _{R}², _{R}³ und Y die oben angegebene Bedeutung haben,
in allgemein üblicher Weise reduziert;
und gegebenenfalls anschließend an die so erhaltenen Verbindungen der Formel (I) eine Säure oder ein Metallsalz addiert.

4) Fungizide Mittel, gekennzeichnet durch einen Gehalt an mindestens einem Azolylvinyldithioacetal der Formel (I) bzw. einem Säureadditions-Salz oder Metallsalz-Komplex von einem Azolylvinyldithioacetal der Formel (I).

5) Verfahren zur Bekämpfung von Pilzen, dadurch gekennzeichnet, daß man Azolylvinyldithioacetale der Formel (I) bzw. deren Säureadditions-Salze oder Metallsalz-Komplexe auf die Pilze und/oder deren Lebensraum ausbringt.

6) Verwendung von Azolylvinyldithioacetalen der Formel (I) bzw. von deren Säureadditions-Salzen und Metallsalz-Komplexen zur Bekämpfung von Pilzen.

7) Verfahren zur Herstellung von fungiziden Mitteln, dadurch gekennzeichnet, daß man Azolylvinyldithioacetale der Formel (I) bzw. deren Säureadditions-Salze oder Metallsalz-Komplexe mit Streckmitteln und/oder oberflächenaktiven Stoffen vermischt.
